# EUROPEAN PATENT SPECIFICATION

(11) **EP 0 922 770 B1**
(45) Date of publication and mention of the grant of the patent: **18.09.2002**
(21) Application number: 98124126.8
(22) Date of filing: 10.10.1995
(51) Int. Cl.: C12P 33/06, A61P 5/24

(54) **A microbiological process for the transformation of 17beta-carboxy substituted 3-oxo-4-azasteroids and the use of such products as inhibitors of the enzyme 5alpha-reductase**
Mikrobiologisches Verfahren zur Transformation von 17-beta-Carboxy-substituierten 3-Oxo-4-Azasteroiden und die Verwendung solcher Produkte als Inhibitoren des Enzyms 5-alpha-Reduktase
Procédé microbiologique de transformation de 3-oxo-4-azasteroides 17-beta-carboxy-substitué et l'utilisation de ces produits comme inhibiteurs de l'enzyme 5-alpha-reductase

(30) Priority: 13.10.1994 IT MI942094
(43) Date of publication of application: 16.06.1999
(62) Divisional of application: 95935926.6
(73) Proprietor: POLI INDUSTRIA CHIMICA S.p.A., 20141 Milano (IT)
(72) Inventor: Poli, Stefano, 20089 Quinto De' Stampi - Rozzano (MI) (IT); Grisenti, Paride, 20089 Quinto De' Stampi - Rozzano (MI) (IT); Magni, Ambrogio, 20089 Quinto De' Stampi - Rozzano (MI) (IT); Leoni, Massimo, 20089 Quinto De' Stampi - Rozzano (MI) (IT)
(74) Representative: Minoja, Fabrizio, Dr.

(56) References cited:
- US-A- 2 969 304
- US-A- 4 379 842
- DODSON, R.M. et al. "Microbiological Transformations. V. 1alpha and 2beta-Hydroxy- lations of C19-Steroids". The Journal of the American Chemical Society, July-Sep. 1960, vol. 82, pages 4026-4033, XP002900434 the whole article.

## Description

The present invention relates to a microbiological process for the transformation of 17β-substituted 4-azaandrostan-3-ones and the use of the biotransformation products as inhibitors of the enzyme 5α-reductase.

The class of molecules known as 4-azasteroids are known to be capable of inhibiting specifically the process of 5α-reduction of testosterone. Up to now, a number of examples of inhibitors of the enzyme 5α-reductase having such a structure are known in literature (Rasmusson, G, H.; Reynolds, G.F.; Utne T.; Jobson R.B.; Primka R.L.; Broocks J. R.; Berman C. J. Med. Chem. 1984, 27, 1690-1701. Rasmusson, G, H.; Reynolds, G. F.; Steimberg, N. G.; Walton, E.; Patel G. F.; Liang T.; Cacsieri M. A.; Cheung A. H.; Broocks J. R.; Berman C. J. Med. Chem. 1986, 29, 2298-2315; EP 155096, EP 538192, EP468012, EP 484094).

Some types of microorganisms are known from literature to be capable of transforming the traditional steroid moiety (Phytochemistry, 1984, 23(10), 2131-2154; Microbial transformation of steroids and alkaloids, Iizuka H., Naito A. University of Tokyo Press, 1967; Yamanè T. et al.. Biotechnology and Bioengineering, 22, 1979, 2133-2141; Sih C.J. et al.. Biochem. Biophys. Acta 38, 1969, 378-379; Dodson R.M. et al.. J. Am. Chem. Soc. 82, 1960, 4026) but we found no evidences from literature of any type of biotransformation on structures such as 17β-carboxy substituted 4-azaandrostan-3-ones as described hereinbelow.

Now it has been found that some microorganisms are capable of monohydroxylating 17β-substituted 4-azaandrostan-3-ones; in particular, using Penicillium sp., Rhizopus, Cunninghamella, mixtures of compounds monohydroxylated at different positions can be obtained. Thus, the object of the present invention is to provide a process for the monohydroxylation of the steroid moiety of compounds of formula (1): 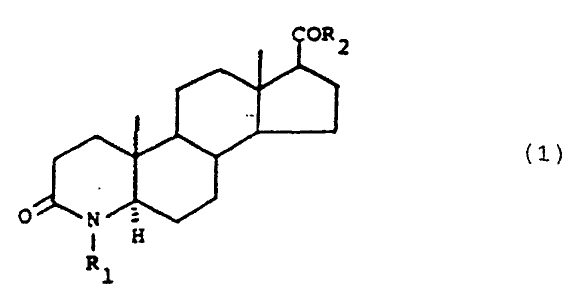 wherein R₁ is hydrogen or a methyl group, R₂ is OH, OR₃, or NHR₃, wherein R₃ is a straight or branched C₁-C₉ alkyl group, a cycloalkyl group containing optionally one o more heteroatoms selected from S, O, N, a C₁₀-C₂₀ polycycloalkyl group, or an aryl group; which process comprises the steps of:
a) culturing a microorganism of the genus Penicillium sp., Rhizopus or Cunninghamella;
b) adding a compound of formula (1): 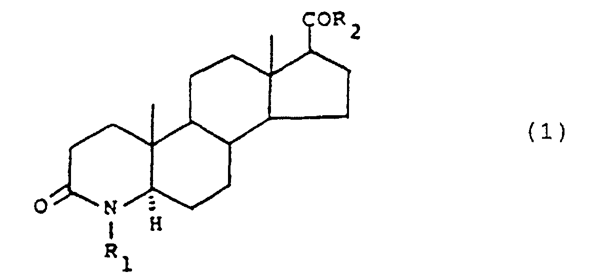 wherein R₁ and R₂ are as defined above; and subsequently incubating;
c) recovering the monohydroxylated derivative obtained in step (b).

Following a general procedure, the hydroxylation reaction is carried out using fungal strains of the genera Penicyllium, Rhizopus or Cunninghamella kept on Sabouraud or PDA (potato-dextrose-agar) slants. These microorganisms are cultured in liquid medium containing one simple sugar as the carbon source. Usually glucose is used, in concentrations from 1 to 50 g/l, but equally suitable to the purpose are molasses in amounts from 5 to 80 g/l. The medium is also added with complex nitrogen sources such as corn steep, peptones (meat or soy peptones), hydrolysated (from casein, albumin or gelatin) and yeast extracts or autolysates. It is useful to combine two or more organic nitrogen sources in such amounts as to reach total concentrations of 2-50 g/l, preferably 5-15 g/l. The medium is complemented with mineral salts, essential for a good growth of the microorganisms, such as sodium and potassium phosphates or diphosphates, magnesium sulfate and calcium, sodium, potassium or magnesium chlorides. The concentrations of these mineral salts usually range from 0.1 to 5 g/l, preferably from 0.2 to 2 g/l. Trace elements, such as iron, zinc, manganese, copper, generally exert a stimulating effect on growth and enzymatic activities. Such trace elements are usually provided in the form of sulfates or chlorides, in concentrations of 1-50 mg/l. The pH of medium is adjusted to a range of 6.00-7.00 (preferably 6.4-6.7) by means of an inorganic base. The culture of the microorganisms is effected at a temperature from 23 to 30°C, preferably from 24 to 28°C for times ranging from 24 to 96 hours (usually from 30 to 48). Once cultures with an efficient vegetative growth have been obtained, the fermentation broth is added directly with the substrate to transform. The latter can be added in the form of either a concentrated solution in a suitable organic solvent, or as a powder, in such an amount as to obtain a final concentration of 100 mg/l to 1 g/l in the reaction mixture. It can be useful to add the substrate to transform in small amounts, or slowly continuously. The hydroxylation reaction is carried out at the same growth temperature for a time ranging from 12 to 120 hours (preferably from 48 to 72 hours), also depending on the final concentration of the substrate to transform.

When the complete transformation of substrate is reached (tlc CHCl₃/CH₃OH 95/5) the reaction mixture is filtered on celite and the filtrate is extracted with CHCl₃. The resulting organic extracts are dried over sodium sulfate, filtered and evaporated under vacuum to give a crude which is subsequently eluted over silica (1/15 ratio); washing subsequently the cake with dichloromethane/methanol 95/5, the mixture of the hydroxylated products is recovered from the filtrate in yields between 60 and 80%. These products can be subsequently recovered by fractional crystallization or by a chromatographic purification over silica (dichloromethane/methanol 95/5 as eluent).

In a preferred embodiment of the invention, the incubation of 17-N-t-butylcarbamoyl-4-azaandrostan-3-one with Penicillium sp. leads to the monohydroxylation of the steroid moiety in 6 and in 15, as shown in the following scheme:

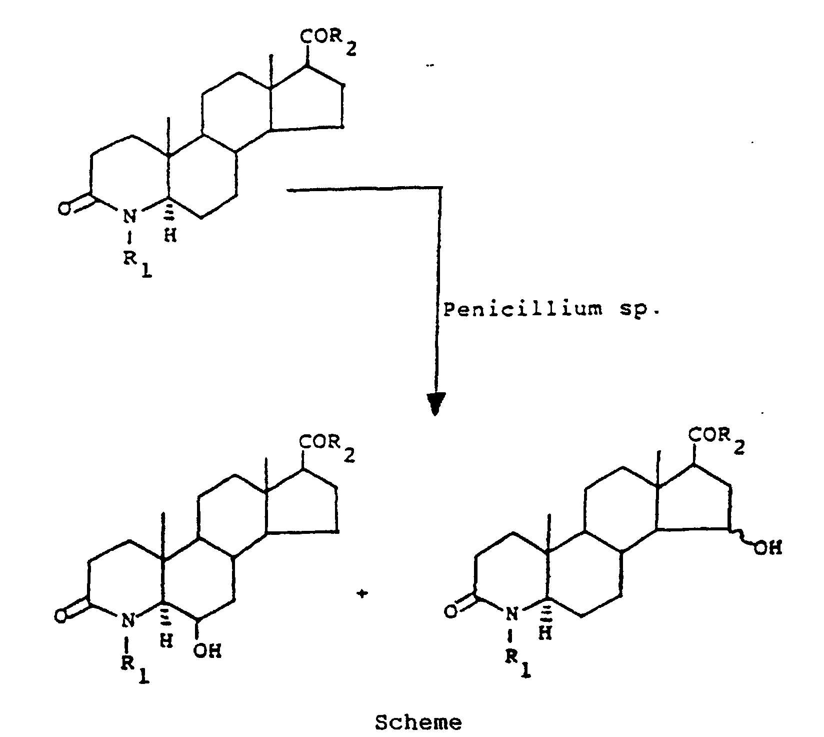

The hydroxylation process can also be carried out using the above mentioned microorganisms immobilized or using crude homogenates obtained from said microorganisms. The related experimental techniques are easy to carry out and known to those skilled in the art (Glass T.L. et al. J. Lipid. Res., 1982, 23, 352; Fukui S. et al. Acta Biotechnol. 1981, 1, 339; Omata T. et al. Eur. J. Microbiol. Biotechnol. 1979, 8, 143; Kim M.N. et al. Biotechnol. Letters 1982, 4, 233; Ohloson S. et al. Eur. J. Appl. Microbiol. Biotechnol. 1979, 7, 103). Moreover, the above mentioned reactions can also be effected using purified enzymes from the same microorganisms. In this case also, the possibility of recovering hydroxylase and using it as biocatalyst in the native state or supported over of a polymer matrix is widely described in literature (Grunwald J. et al. J. Am. Chem. Soc. 1986,108, 6732; Snjider-Kambers A.M. et al. Recl. Trav. Chim. Pays-Bas 1991, 110, 226; Santaniello E. et al. Chem. Rev. 92, 1992, 1071-1140).

The following example further illustrate the invention.

### Example

### Hydroxylation of 17-t-butylcarbamoyl-4-azaandrostan-3-one with Penicillium sp.

The fungus is kept on "EMMON'S modification of SABOURAUD'S AGAR" slants, in thermo-stat at a temperature of 26-27°C for 6-7 days until obtaining an abundantly sporulated mycelium. Subsequently the slants can be kept at 5°C for 2-3 months. The vegetative culture is carried out in the same medium as that used for the conservation, free from agar, distributed into 300 ml flasks at the rate of 50 ml/flask. In order to carry out the inoculation, 1.0-1.5 cm² of mycelium for each flask to inoculate are withdrawn sterilely from a slant by means of a spatula. The felt is finely ground in Potter with about 1 ml of saline solution and the resulting suspension is distributed into the flasks, which are incubated at 27°C on a rotary shaker at 140 rpm for 48 hours. The primary vegetative culture is used to inoculate a medium having the following composition:

| Ingredients | Concentration (g/l) |
|---|---|
| Anhydrous dextrose | 5.000 |
| Atomized Corn Steep | 2.000 |
| Soy peptone | 1.000 |
| Urea | 0.300 |
| Monobasic potassium phosphate | 1.000 |
| Magnesium sulfate.7H₂O | 0.300 |
| Iron sulfate.7H₂O | 0.005 |
| Zinc sulfate.7H₂O | 0.005 |
| Manganese sulfate.H₂O | 0.001 |
| Copper sulfate.5H₂O | 0.001 |

The final pH is adjusted to 6.5 with 1N KOH, distributed into 300 ml flasks at the rate of 50 ml/flask and sterilised at 121°C for 20 minutes. The flasks inoculated with 10% of vegetative culture are incubated at a temperature of 27°C (140 rpm) for 24 hours. After this time, in case large pellets or mycelium growth confluent into a single block are present, homogenization in Blendor is effected for some seconds. The finely dispersed mycelium is added with 0.35 ml/flask of a solution of 42 mg/l of 17β-t-butylcarbamoyl-4-azaandrostan-3-one in ethanol (final concentration in the flask 270 mg/l of substrate) and the whole is incubated again in the above defined conditions. After 24 hours each flask is added with a further 0.7 ml of the same solution, incubating again at 27°C for a further 72 hours. At the end of the transformation the reaction mixture is filtered on celite and the filtrate is extracted with CHCl₃ (3 times for an equal volume of the aqueous phase). The organic extracts are dried over sodium sulfate, filtered and evaporated to dryness to give a crude which is subsequently eluted on silica (1/15 ratio to the crude): subsequent washings with CH₂Cl₂/MeOH 95/5 allow to recover in 90% yields the mixture of monohydroxylated products which are subsequently separated by crystallization from ethyl acetate. Three products have been obtained having the following spectrum characteristics:
1st product:
   ¹H-NMR (500 MHz; CDCl₃; diagnostic signals) δ: 0.7 (s, 3H), 0.95 (s, 3H), 1.45 (s, 9H), 2.4 (m, 2H), 2.88 (d, 1H), 3.55 (m, 1H), 5.1 (s, 1H), 6.8 (s, 1H, exchangeable);
   MS (m/e): 390.30 (m+), 375.30 (m-15), 372.25 (m-18), 357.40 (m-33), 318.30 (m-72), 290.25 (m-100);
   assignable to 17β-t-butylcarbamoyl-6a-hydroxy-4-azaandrostan-3-one;
2nd and 3rd product:
   ¹H-NMR (500 MHz; CDCl₃; diagnostic signals) δ: 0.92-1.04 (dd, 6H), 1.37 (d, 9H), 2.42 (m, 2H), 3.05 (m, 1H), 4.3 and 5.05 (2m, 1H total), 5.22 (s, 1H, exchangeable), 5.5 (s, 1H, exchangeable);
   MS (m/e): 390.30 (m+), 372.25 (m-18), 357.25 (m-33), 331.25 (m-59), 314.20 (m-73), 290.30 (m-100);
   assignable to 17β-t-butylcarbamoyl-15a-hydroxy-4-azaandrostan-3-one and to 17β-t-butylcarbamoyl-15β-hydroxy-4-azaandrostan-3-one.

The three monohydroxylated products obtained by biotransformation of the azasteroid moiety using Penicillium sp. have biological activity on the enzyme 5α-reductase. For this purpose, the enzymatic in vitro method described in WO/9413691, published on June 23, 1994 has been used. Unexpectedly, a strong inhibiting activity on the enzyme 5α-reductase was evidenced for the products 2 and 3, their concentration inhibiting by 50% the enzymatic activity (IC₅₀.) being superimposable to that of Finasteride, used as the control product, whereas product 1 turned out to be poorly active.

| | IC₅₀ (nM) |
|---|---|
| Product 1 | 195 |
| Product 2 | 35 |
| Product 3 | 35 |
| Finasteride | 37 |

Therefore, the present invention also provides the use of the monohydroxylated compounds as therapeutical agents, particularly for the preparation of a medicament having inhibiting activity on testosterone 5α-reductase.

The present invention also provides pharmaceutical compositions for the oral; parenteral and topical administrations, containing the above mentioned compounds together with conventional, pharmaceutically acceptable carriers and excipients.

Examples of oral pharmaceutical compositions are tablets, capsules, sachets and suspensions; examples of parenteral compositions are freeze-dried ampoules or sterile suspensions; examples of topical compositions are creams, ointments, gels, aerosols or foams.

The compositions of the invention are prepared with conventional methods, as for example those described in Remington's Pharmaceutical Sciences Handbook, Mack Pub. XVII Ed. N.Y., U.S.A.

The daily dosage and mode of administration will be decided by the physician, depending on the disease to treat, the severity of the symptoms and the conditions of the patient (age, sex and weight).

## Claims

1. A process for the monohydroxylation of the steroid moiety of compounds of formula (1): 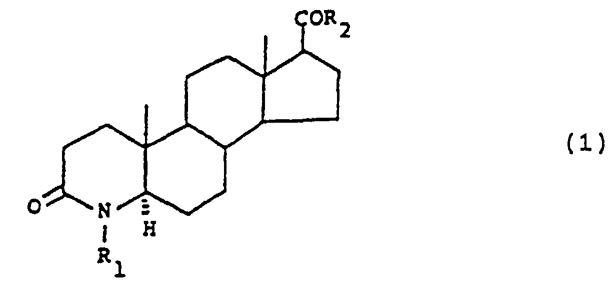 wherein R₁ is hydrogen or a methyl group, R₂ is OH, OR₃, or NHR₃, wherein R₃ is a straight or branched C₁-C₉ alkyl group, a cycloalkyl group containing optionally one or more heteroatoms selected from S, O, N, a C₁₀-C₂₀ polycycloalkyl group or an aryl group; which process comprises the steps of:
a) culturing a microorganism of genus Penicillium, Rhizopus or Cunninghamella;
b) adding a compound of formula (1): 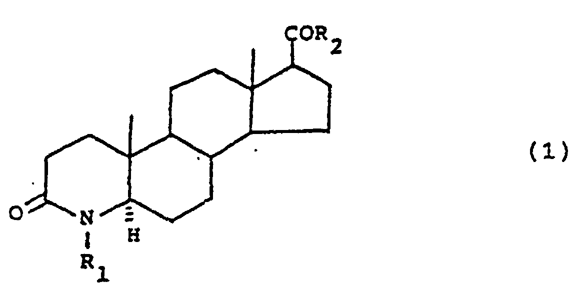 wherein R₁ and R₂ are as defined above; and subsequently incubating;
c) recovering the resulting monohydroxylated derivative.

2. A process according to claim 1, wherein said microorganism is Penicillium.

3. A process according to either of claims 1 and 2, wherein in compound of formula 1 R₂ is a tert-C₄H₉-NH- group.

4. A monohydroxylated compound obtainable by the process of any one of claims 1, 2 and 3.

5. A compound according to claim 4, of formula: 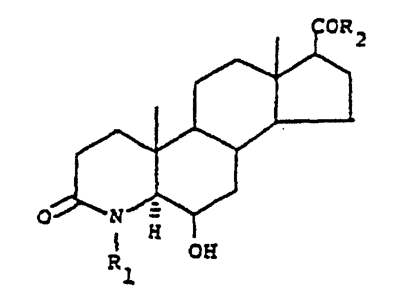 wherein R₁ and R₂ are as defined above.

6. A compound according to claim 4, of formula: 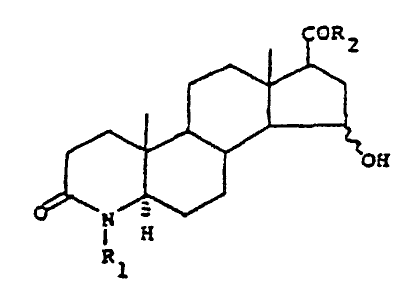 wherein R₁ and R₂ are as defined above.

7. A compound of any one of claims 4 to 6 for use as a therapeutical agent.

8. The use of a compound of any one of claims 4 to 6 for the preparation of a medicament having inhibiting activity on testosterone 5α-reductase.

9. A pharmaceutical composition containing a compound of any one of claims 4 to 6 as active ingredient, in admixture with pharmaceutically acceptable carriers and excipients.

## Patentansprüche

1. Verfahren für die Monohydroxylierung des steroiden Anteils von Verbindungen der Formel (1): 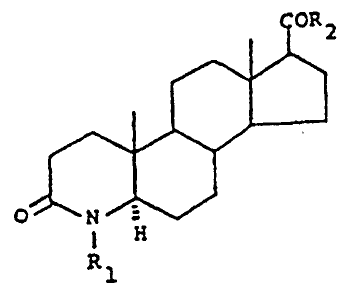 worin R₁ Wasserstoff oder eine Methylgruppe ist, R₂ = OH, OR₃ oder NHR₃, worin R₃ eine gerade oder verzweigte C₁ - C₉ Alkylgruppe, eine Cycloalkylgruppe, die wahlweise ein oder mehrere Heteroatome enthält, ausgewählt von S, O, N, einer C10 - C20 Polycycloalkylgruppe oder einer Arylgruppe; wobei das Verfahren besteht aus den Stufen:
a) Züchtung eines Mikroorganismus vom Genus Penicillium, Rhizopus oder Cunninghamella;
b) Hinzufügung einer Verbindung der Formel (1): 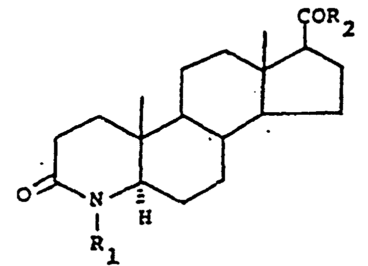 worin R₁ und R₂ wie vorstehend definiert sind; und anschließendes Inkubatieren;
c) Rückgewinnung des resultierenden monohydroxilierten Derivats.

2. Verfahren nach Anspruch 1, bei welchem der Mikroorganismus Pennicilium ist.

3. Verfahren nach Anspruch 1 oder 2, bei welchem in der Verbindung der Formel 1 R₂ eine tertiäre C₄H₉-NH-Gruppe ist.

4. Monohydroxilierte Verbindung, die durch das Verfahren nach einem der Ansprüche 1, 2 und 3 zu erhalten ist.

5. Verbindung nach Anspruch 4 mit der Formel: 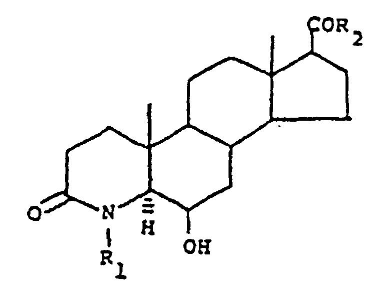 worin R₁ und R₂ wie vorstehend definiert sind.

6. Verbindung nach Anspruch 4 mit der Formel: 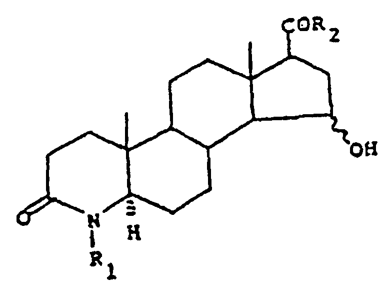 worin R₁ und R₂ wie vorstehend definiert sind.

7. Verbindung nach einem der Ansprüche 4 bis 6 zur Verwendung als ein therapeutisches Mittel.

8. Verwendung einer Verbindung nach einem der Ansprüche 4 bis 6 für die Herstellung eines Arnzeimittels mit einer hemmenden Aktivität gegenüber Testosteron 5α-Reduktase.

9. Pharmazeutische Mischung, die eine Verbindung nach einem der Ansprüche 4 bis 6 als aktiven Bestandteil enthält, vermischt mit pharmazeutisch zulässigen Trägersubstanzen und Arzneimittelträgern.

## Revendications

1. Un procédé pour la monohydroxylation de la portion stéroidique de composés de formule (1) : 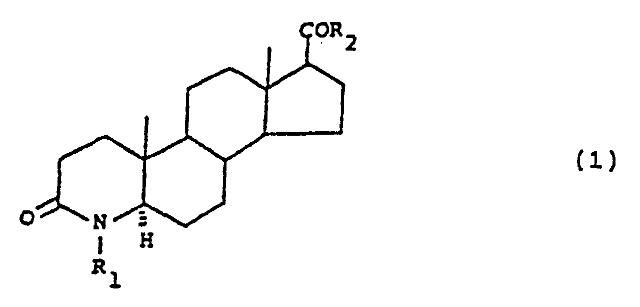 dans laquelle R₁ est l'hydrogène ou un groupe méthyle, R₂ est OH, OR₃ ou NHR₃, où R₃ est un groupe alkyle en C₁-C₉ linéaire ou ramifié, un groupe cycloalkyle contenant facultativement un ou plusieurs hétéroatomes choisis parmi S, O, N, un groupe polycycloalkyle en C₁₀-C₂₀ ou un groupe aryle ; lequel procédé comprenant les étapes suivantes :
a) cultiver un microorganisme du genre Penicillium, Rhizopus ou Cunninghamella ;
b) ajouter un composé de formule (1) : 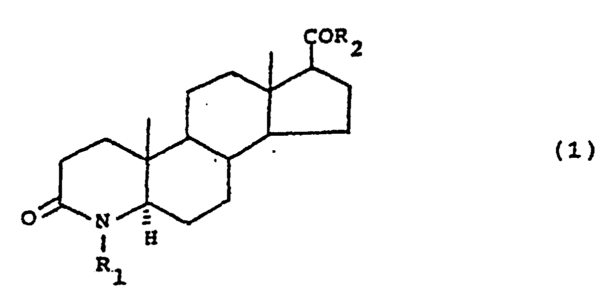 dans laquelle R₁ et R₂ sont tels que définis ci-dessus ; puis mettre en incubation ;
c) recueillir le dérivé monohydroxylé résultant.

2. Un procédé selon la revendication 1, dans lequel ledit microorganisme est du genre Pénicillium.

3. Un procédé selon l'une ou l'autre des revendications 1 et 2, dans lequel, dans le composé de formule (1), R₂ est un groupe *tert*-C₄H₉-NH-.

4. Un composé monohydroxylé pouvant être obtenu par le procédé de l'une quelconque des revendications 1, 2 et 3.

5. Un composé selon la revendication 4, de formule : 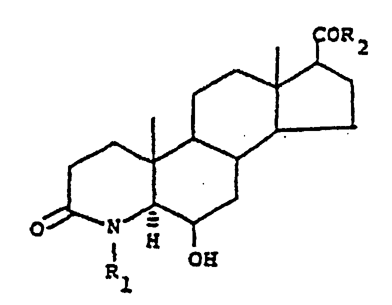 dans laquelle R₁ et R₂ sont tels que définis ci-dessus.

6. Un composé selon la revendication 4, de formule : 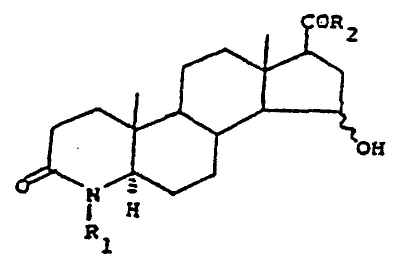 dans laquelle R₁ et R₂ sont tels que définis ci-dessus.

7. Un composé de l'une quelconque des revendications 4 à 6 à utiliser comme agent thérapeutique.

8. L'utilisation d'un composé de l'une quelconque des revendications 4 à 6 pour la préparation d'un médicament ayant une activité inhibitrice de testostérone-5_{α}-réductase.

9. Une composition pharmaceutique contenant un composé de l'une quelconque des revendications 4 à 6 comme ingrédient actif en mélange avec des supports et excipients pharmaceutiquement acceptables.
